# EUROPÄISCHE PATENTSCHRIFT

(11) **EP 4 696 247 B1**
(45) Veröffentlichungstag und Bekanntmachung des Hinweises auf die Patenterteilung: **23.09.2026**
(21) Anmeldenummer: 24194591.4
(22) Anmeldetag: 14.08.2024
(51) Int. Cl.: A61B 17/00

(54) **BERGEBEUTELINSTRUMENT ZUM BERGEN VON RESEZIERTEM GEWEBE BEI EINER ENDOSKOPISCHEN RESEKTION, BERGEBEUTEL, VERFAHREN ZUR HERSTELLUNG EINES BERGEBEUTELS UND VERFAHREN ZUR HERSTELLUNG EINES BERGEBEUTELINSTRUMENTS**
RESCUE BAG INSTRUMENT FOR RECOVERING RESECTED TISSUE IN ENDOSCOPIC RESECTION, RESCUE BAG, METHOD FOR PRODUCING A RESCUE BAG, AND METHOD FOR PRODUCING A RESCUE BAG INSTRUMENT
INSTRUMENT DE POCHE DE MONTAGNE POUR RÉCUPÉRER UN TISSU RÉSÉQUÉ LORS D'UNE RÉSECTION ENDOSCOPIQUE, SAC DE MONTAGNE, PROCÉDÉ DE FABRICATION D'UN SAC DE MONTAGNE ET PROCÉDÉ DE FABRICATION D'UN INSTRUMENT DE POCHE DE MONTAGNE

(43) Veröffentlichungstag der Anmeldung: 18.02.2026
(73) Patentinhaber: Urotech GmbH, 83101 Rohrdorf-Achenmühle (DE)
(72) Erfinder: MÜLLER, Felix, 83253 Rimsting (DE)
(74) Vertreter: Hofstetter, Schurack & Partner

(56) Entgegenhaltungen:
- DE-U1- 202011 051 747
- US-A- 5 352 184
- US-B2- 10 154 833
- US-B2- 11 642 113

## Beschreibung

Die vorliegende Erfindung betrifft einen Bergebeutel zum Bergen von reseziertem Gewebe bei einer endoskopischen Resektion, ein Bergebeutelinstrument mit dem Bergebeutel, ein Verfahren zur Herstellen eines Bergebeutels für das Bergebeutelinstrument und ein Verfahren zur Herstellung des Bergebeutelinstruments.

Bei einer endoskopischen Operation, insbesondere einer transurethralen Resektion (durch die Harnröhre) beispielsweise eines Harnblasentumors mittels eines Endoskops beziehungsweise Resektoskops, stellt sich die Herausforderung, ein Resektat in einem Stück ("en bloc") aus dem Körper zu entfernen. Gewöhnlich ist das Resektat ein Stück eines NMIBCs (Non-Muscle-Invasive Bladder Cancer), der nach der Resektion durch beispielsweise aus dem Körper entfernt werden muss. Eine erneute Fragmentierung des resezierten Tumors kann hierbei zu einem erhöhten Risiko einer Tumorreimplantation und einem "Cell Seeding" führen, das heißt einer Ausbreitung von Tumorzellen. Gegenwärtig lassen sich Resektate, die einen Durchmesser von größer als 2 cm aufweisen, nicht en bloc bergen, das heißt nicht in einem Stück bergen. Im Stand der Technik werden in solch einem Fall Schneidvorrichtungen verwendet, die das Resektat in kleinere Stücke schneiden können, die jedoch aufwendig sind und die Gefahr des Cell Seedings bergen.

Medizinische Instrumente zum Bergen von bei endoskopischen Operationen zu entfernendem Körpergewebe, beispielsweise aus einer mit Flüssigkeit gefüllten Harn- oder Gallenblase, sind in unterschiedlichen Ausführungsformen bekannt.

In einer bekannten Ausführungsform ist ein Beutel ("Bergebeutel") mittels eines Bügels aus einem federelastischen Material am distalen Ende eines Schafts des medizinischen Instrumentes gehalten. Im Auslieferungszustand des Instrumentes ist der Beutel beispielsweise aufgerollt und befindet sich mit dem zusammengedrückten Bügel in einem den Schaft umgebenden Hüllrohr. In diesem Zustand wird der Schaft durch einen Trokar eingeführt. Wird das Hüllrohr auf dem Schaft in Richtung zum proximalen Ende des Schaftes zurückgezogen, so gelangen der Bügel und der an ihm befestigte Beutel aus dem distalen Ende des Hüllrohrs, sodass sich der Beutel entrollen kann und sich der Bügel unter Ausbildung einer Einfüllöffnung am Beutel auseinanderfaltet. Nach dem Einfüllen des zu bergenden Materials beziehungsweise des Resektats in den Beutel kann dieser durch Ziehen an dem Bügel geschlossen werden, wobei der Bügel nach und nach wieder zusammengedrückt wird und in das Hüllrohr gezogen wird. Ein weiterer möglicher Verschluss des Beutels kann mittels einer sogenannten "Tabaksbeutelnaht" erreicht werden. Hierbei wird eine in einem Kanal des Beutels geführte Fadenschlaufe zusammengezogen, wobei der Kanal den Beutel im Bereich seiner Einfüllöffnung umgibt und wobei der Beutel vom federelastischen Bügel abgerissen und die Einfüllöffnung des Beutels verschlossen wird. Jedoch kann bei einem Herausziehen des Beutels ein Platzen auftreten, das der Beutel in das Hüllrohr gezogen wird und dadurch abgeschlossen wird. Eingeschlossenes Fluid (Gas und/oder Flüssigkeit) kann so nicht mehr aus dem Beutel heraustreten, was zum Platzen des Bergebeutels führen kann

Beispielsweise offenbart die Druckschrift DE 42 20 785 C2 einen Laparoskopiebeutel aus einem flexiblen Material mit an der Öffnung angebrachten Zugschnüren, insbesondere für die Verwendung in einem Trokar, dadurch gekennzeichnet, dass der Beutel aus einem Arbeitsteil besteht, an den sich ein Schlauchteil anschließt, welches so bemessen ist, dass es in gefülltem Zustand durch den Trokar entnommen werden kann. Eine Laparoskopie (Schlüsselloch-Chirurgie) ist ein hierbei ein minimalinvasives chirurgisches Verfahren, bei dem kleine Schnitte (Ports) in eine Bauchdecke gemacht werden, durch die dünne Instrumente und eine Kamera eingeführt werden können.

Ferner offenbart beispielsweise die Druckschrift US 5 785 677 A einen Laparoskopiebeutel aus einem flexiblen Material, der an seiner Öffnung oder Mündung mit Zugschnüren versehen ist, insbesondere zur Verwendung in einem Trokar, und bei dem der Beutel aus einem Arbeitsabschnitt besteht, an dem ein schlauchförmiges Teil oder eine schlauchförmige Verlängerung angebracht ist oder der mit einer schlauchförmigen Verlängerung versehen ist.

Zudem offenbart die Druckschrift DE 20 2011 051 747 U1 ein medizinisches Instrument mit einem Bergebeutel zum Bergen von bei einer Operation, insbesondere einer endoskopischen Operation, aus einem zu operierenden Körper zu entfernendem Material, der mittels eines Bügels an einem Schaft gehalten ist, wobei die Zuschnittform des Bergebeutels in distale Richtung ausgerichtet ist, wobei der flach ausgelegte Bergebeutel bezogen auf die Längsachse des Schaftes über den Bügel distal vorsteht.

Ferner beschreibt die Druckschrift US 5 352 184 A1 einen Behälter zur Aufnahme und Entnahme von Körperproben, die Druckschrift US 11 642 113 B2 beschreibt ein Interventionsgerät zur Probenentnahme, und die Druckschrift US 10 154 833 B2 beschreibt eine Probenentnahmevorrichtung mit einem Beutelanschlag.

Jedoch ergibt sich das Problem, dass beim Schließvorgang des Bergebeutels Beutelmaterial in den Tubus gezogen werden kann, sich eine Beuteltiefe reduzieren kann und das in der Flüssigkeit schwebende Resektat aus dem Bergebeutel herausgedrückt werden kann (im Folgenden "Trampolineffekt" genannt). Ferner gibt es Verbesserungsbedarf in Bezug auf eine Handhabung des Bergebeutels.

Somit ergibt sich die Aufgabe, ein verbessertes Bergebeutelinstrument bereitzustellen, das bei einer endoskopischen Resektion von Gewebe verwendbar ist und ein Einfangen eines in Flüssigkeit befindlichen Resektats vereinfacht.

Diese Aufgabe wird erfindungsgemäß durch die Gegenstände der unabhängigen Patentansprüche gelöst. Vorteilhafte Ausführungen der Erfindung sind Gegenstand der abhängigen Patentansprüche und der Beschreibung.

Eine Kernidee der Erfindung ist es beispielsweise, dass in einem Bergebeutel des Bergebeutelinstruments Abflusslöcher angebracht werden, die ein Austreten von Fluid (Gas und/oder Flüssigkeit) aus dem Bergebeutel bei einem Zuziehen des Bergebeutels ermöglichen. Ferner ist es eine weitere Kernidee der Erfindung, die Zuschiebemechanik durch eine feste Gesamtlänge des Bergebeutelinstruments und eine veränderte ziehharmonikaartige Auffädelung des Beutelmaterials auf dem Halteseil zu verbessern.

Gemäß einem Aspekt der Offenbarung ist ein Bergebeutel zum Bergen von reseziertem Gewebe bei einer endoskopischen Resektion bereitgestellt, wobei der Bergebeutel beutelförmig mit einem Beutelgrund und einer dem Beutelgrund gegenüberliegenden verschließbaren Fangöffnung ausgebildet ist, der Bergebeutel eine Vielzahl von Abflusslöchern mit einem vorgegebenen Durchmesser aufweist, die dazu geeignet sind, Fluid aus dem Bergebeutel nach außen austreten zu lassen, der Bergebeutel an einem proximalen Ende des Bergebeutels eine Verbindungslasche aufweist, die dazu eingerichtet ist, an dem Hüllrohr befestigt zu werden, der Bergebeutel Durchtrittsöffnungen aufweist, die um die Fangöffnung herum angeordnet sind und die dazu eingerichtet sind, ein Halteseil hindurchzuführen, an dem der Bergebeutel im Bereich der Schlinge haltbar ist, und wobei der Bergebeutel an einem dem proximalen Ende gegenüberliegenden distalen Ende einen schlauchförmigen Schlauchabschnitt aufweist, durch den das Halteseil in Umfangsrichtung der Fangöffnung hindurchgeführt ist.

Mit anderen Worten ist ein Bergebeutel für ein Bergebeutelinstrument zum Bergen von Gewebe bereitgestellt, das beispielsweise bei einer endoskopischen, insbesondere transurethralen, Resektion abgetragen werden kann. Die transurethrale Resektion ist hierbei eine urologische Operationstechnik, bei der erkranktes Gewebe aus beispielsweise einer Harnblase oder einer Prostata abgetragen werden kann. Die Operation kann endoskopisch durch die Harnröhre ohne äußeren Einschnitt erfolgen und kann unter Vollnarkose oder mittels einer Spinalanästhesie einschließlich einer Nervus-obturatorius-Blockade durchgeführt werden. Das Bergebeutelinstrument kann jedoch auch in Verbindung mit einem Resektoskop, einem Trokar, einem Endoskop, einem Rektoskop und/oder einem Zytoskop verwendet werden.

Ein Trokar ist ein medizinisches Instrument, mit dem scharf oder stumpf ein Zugang zu einer Körperhöhle (z. B. Bauchraum, Brustraum) geschaffen und durch ein Rohr (Tubus) offengehalten wird. Trokare können in der minimalinvasiven Chirurgie zum Setzen von Arbeits- und Sichtkanälen eingesetzt werden.

Ein Endoskop ist ein medizinisches Instrument, das zur Betrachtung des Inneren von Hohlorganen oder Körperhöhlen eingesetzt werden kann. Es kann aus einen dünnen, flexiblen Schlauch mit einer Lichtquelle und einer Kamera an einem distalen Ende des Endoskops umfassen. Je nach Verwendungszweck können Endoskope unterschiedliche Ausprägungen aufweisen, die mit unterschiedlichen Namen wie beispielsweise Rektoskop, Zytoskop, Resektoskop usw. bezeichnet sein können.

Ein Rektoskop ist beispielsweise ein spezielles Endoskop, das zur Untersuchung eines Mastdarms (Rektum) verwendet werden kann. Es kann beispielsweise etwa 10-20 Zentimeter lang sein und einen Durchmesser von etwa 1-2 Zentimetern aufweisen. Mit einem Rektoskop können Chirurgen beispielsweise Polypen, Tumore oder andere Anomalien im Mastdarm erkennen und behandeln.

Ein Zytoskop ist ein weiteres spezielles Endoskop, das zur Untersuchung einer Harnblase eingesetzt werden kann. Es kann durch die Harnröhre in die Harnblase eingeführt werden und ermöglicht eine Betrachtung der Innenwand der Harnblase. Zytoskope können verwendet werden, um beispielsweise Harnblasenkrebs oder andere Erkrankungen der Blase zu diagnostizieren.

Ein Resektoskop ist ein spezielles Endoskop, das verwendet werden kann, um Gewebewachstum in Hohlorganen wie der Blase, der Prostata oder dem Uterus zu entfernen. Es kann einen dünnen, flexiblen Schlauch mit einer Lichtquelle, eine Kamera und eine elektrische Schlinge an dem distalen Ende umfassen. Mit einem Resektoskop können Chirurgen Gewebe durch die Schlinge fassen, schneiden und/oder abtragen, während sie den Vorgang unter Sicht durch die Optik überwachen können. Das entfernte Gewebe kann zur Untersuchung an ein Labor geschickt werden, um beispielsweise sicherzustellen, dass ein Tumor vollständig entfernt wurde. Resektoskope können bei einer Behandlung von gutartigen Wachstum wie Polypen oder bei der Entfernung von Krebsvorstufen eingesetzt werden. Sie können auch zur Behandlung von Blasentumoren, Prostatavergrößerungen und anderen Erkrankungen verwendet werden.

Eine transurethrale Resektion kann beispielsweise mit Hilfe eines Resektoskops durchgeführt werden. Ein Resektoskops kann als Rückspülresektoskop beispielsweise einen in der Harnröhre atraumatisch (ohne die Harnröhre zu verletzen) ruhenden Außenschaft mit einem Kanal für eine Flüssigkeitszufuhr und einem Kanal für eine Absaugung umfassen. Ein Innenschaft des Rückspülresektoskop kann eine Optik samt einer Transporteinrichtung für eine Längsbewegung einer Resektionsschlinge umfassen. An die Optik können beispielsweise eine Lichtquelle sowie eine Videokamera für eine Bildgebung angeschlossen werden.

Für die transurethrale Resektion kann beispielsweise eine Drahtschlinge verwendet werden, durch die ein elektrischer Strom hindurchgeleitet werden kann. Damit kann erkranktes Gewebe in der Harnblase oder Prostata schichtweise abgetragen werden, das in der Regel histologisch untersucht werden soll. Auftretende Blutungen können elektrisch verödet werden (Kauterisierung). Während der Operation kann eine Spülflüssigkeit über das Resektoskop eingebracht und kontrolliert abgesaugt werden, dadurch kann eine konstante Blasenfüllung und gute Sicht gewährleistet werden. Am Ende der Operation wird das resezierte Gewebe ausgespült und nach ausreichender Blutstillung ein Spülkatheter eingelegt.

Das Bergebeutelinstrument umfasst den beutelförmigen Bergebeutel, der für eine Entfernung von reseziertem Gewebe verwendet werden kann. "Beutelförmig" bedeutet hier beispielsweise sackförmig beziehungsweise annähernd kegelstumpfförmig, wobei der Bergebeutel die Mantelfläche und die Deckfläche eines nach unten sich verjüngenden Kegelstumpfes ausbildet. Die Grundfläche des Kegelstumpfes wird hierbei durch die Fangöffnung gebildet. Natürlich ist dies eine Idealisierung, da der Bergebeutel aus einem flexiblen Kunststoffmaterial ausgebildet sein kann, bei dem Ecken und Kanten durch den Herstellvorgang abgerundet sein können. Zudem kann der Durchmesser entlang der Höhe des Kegelstumpfes nicht nur sich linear verjüngen, sondern kann auch zylinderförmige Abschnitte oder Abschnitte mit sich erweiterndem Durchmesser umfassen. Der Bergebeutel ist durch die Deckfläche des Kegelstumpfes abgeschlossen und die Deckfläche und der benachbarte Teil der Mantelfläche bildet somit ein geschlossenes Ende der Sackbeziehungsweise Kegelstumpfform und dieses geschlossene Ende wird auch als Beutelgrund bezeichnet. Mit anderen Worten, der Beutelgrund umfasst das geschlossene Ende der Sack- beziehungsweise Kegelstumpfform, und ist somit ein geschlossener Bergebeutelabschnitt, der ein maximales Volumen (also ein inneres Volumen in dem Beutelgrund in einem Zustand maximaler Größe des Bergebeutels) aufweist, in dessen Volumen das Resektat einbringbar ist. Der Beutelgrund wird auch als unteres Ende des Bergebeutels bezeichnet. Dem Beutelgrund liegt die Fangöffnung gegenüber, die mittels eines Halteseils verschließbar beziehungsweise öffenbar ist. Die Fangöffnung bildet das somit das obere Ende des Bergebeutels.

Der Bergebeutel weist zudem neben der Fangöffnung eine Vielzahl von Abflusslöchern auf, die einen vorgegebenen Durchmesser aufweisen und die dazu geeignet sind, ein in dem Bergebeutel befindliches Fluid (ein in dem Bergebeutel enthaltenes Gas und/oder eine in dem Bergebeutel enthaltene Flüssigkeit) aus dem Bergebeutel nach außen austreten zu lassen. Mit anderen Worten, die Abflusslöcher weisen eine derartige Größe auf, dass Gewebestücke mit einer Größe, die über dem vorgegebenen Durchmesser der Abflusslöcher liegt, zurückgehalten werden und somit auch kleine Resektate mit einem durchschnittlichen Durchmesser von beispielsweise 3 mm, 2mm oder 1 mm zurückgehalten werden können, wobei dennoch das Fluid aus dem Inneren des Bergebeutels nach außen abfließen kann. Der Bergebeutel ist einem flüssigkeitsundurchlässigen beziehungsweise gasdichten Material ausgebildet, sodass das in dem Bergebeutel enthaltene Fluid nur durch die Fangöffnung beziehungsweise durch die Abflusslöcher abfließen kann. Dadurch, dass das Fluid durch die Abflusslöcher abfließen kann, ist bei einem Verschließen des Bergebeutels die Ausstoßneigung beziehungsweise der Trampolineffekt deutlich verringert und das Resektat kann sicher eingefangen werden.

Vorzugsweise ist der Bergebeutel des Bergebeutelinstruments aus einem stabilen Kunststoff hergestellt, wobei es besonders bevorzugt ist, falls der Bergebeutel aus einem hochreißfesten, flüssigkeitsdichten, elastischen und/oder thermoplastischen Kunststoff hergestellt ist. Insbesondere ist es bevorzugt, dass der Bergebeutel des Bergebeutelinstruments aus Polyurethan hergestellt ist.

Eine allgemeine Vorgehensweise bei einer Verwendung des Bergebeutelinstruments ist nachstehend geschildert. Nach einer Resektion von beispielsweise Tumorgewebe bei einer transurethralen Resektionsoperation mittels eines Endoskops kann der Benutzer des Bergebeutelinstruments dieses durch einen Arbeitskanal des verwendeten Endoskops beziehungsweise Trokars einführen. Sobald das distale Ende des Bergebeutelinstruments in dem Kamerabild erscheint, kann der Benutzer dieses Kamerabild verwenden, um den Bergebeutel zu dem Resektat zu navigieren.

Falls dies notwendig ist, kann der Benutzer die Fangöffnung dazu verwenden, um einen Teil des Resektats zu greifen und dieses in eine vorteilhafte Position in Bezug auf das Kamerabild und die Fangöffnung zu bringen, insbesondere über die Fangöffnung in Schwerkraftrichtung zu bringen. Öffnet der Benutzer in dieser Position die Fangöffnung wieder, kann das Resektat in den Bergebeutel fallen. Der Benutzer kann schließlich den Bergebeutel schließen, um das Resektat einzuschließen. Der Benutzer kann also das Bergebeutelinstrument auch als Manipulator einsetzen, um Gewebe zu greifen und zu manipulieren.

Im Stand der Technik sind Bergebeutel gewöhnlich mit der Aufstellschlinge fest verbunden, was zum Nachteil beim Einfangvorgang wird. Dadurch wird beim Schließvorgang des Bergebeutels Beutelmaterial in den Tubus gezogen, was wiederrum die Beuteltiefe reduziert und das leicht schwebende Resektat aus dem Bergebeutel herausdrückt ("Trampolineffekt"). Somit eignen sich Bergebeutel des Stands der Technik nicht zum Greifen von Gewebe.

Die Abflusslöcher des Bergebeutels der vorliegenden Offenbarung ermöglichen jedoch ein Schließen des Bergebeutels mit deutlich verringerter Ausstoßneigung, da enthaltenes Fluid durch die Abflusslöcher abfließen kann.

Zudem ermöglicht die feste Länge des Halteseils in Bezug auf den Griffabschnitt der Handkinematik, dass bei einem Zuschieben des Bergebeutels dieser nicht aus dem Kamerabild verschwindet. Im Stand der Technik wird ein Schließmechanismus eines Bergebeutels durch ein Zurückziehen der Bergebeutelschlinge realisiert. Dadurch bewegt sich die Schlinge samt verbundenem Bergebeutel in proximale Richtung und in den Tubus hinein. Dies vergrößert die Entfernung zwischen Bergebeutel und Resektat und verkompliziert den Einfangprozess enorm, v. a. in einer mit Flüssigkeit gefüllten Umgebung.

Danach kann der Benutzer das Bergebeutelinstrument durch den Trokar herausziehen. Hierbei wird der Bergebeutel zusammengedrückt und muss gegen einen Widerstand herausgezogen werden. Dadurch wird ein weiteres Mal das Volumen des Bergebeutels verringert und die Abflusslöcher können auf besonders vorteilhafte Weise ein Platzen des Bergebeutels verhindern, da enthaltenes Fluid einfach abfließen kann. Dadurch, dass am Beutelgrund keine Abflusslöcher vorhanden sind, kann während des Herausziehens ein Cell Seeding verhindert werden.

Sollte das Resektat für den Trokar zu groß sein, kann der Trokar zusammen mit dem Bergebeutelinstrument herausgezogen werden. Auch hierbei können die Abflusslöcher auf besonders vorteilhafte Weise ein Platzen des Bergebeutels verhindern, da enthaltenes Fluid einfach abfließen kann. Herkömmliche Bergebeutel eignen sich jedoch nicht dazu, das gefangene und umschlossene Resektat, welches aufgrund der Größe weder durch den Innen- noch Außenschaft des Resektoskops oder Zystoskop passt, durch die Harnröhre zu bergen.

Nach dem Herausziehen kann die Fangöffnung einfach geöffnet werden, das Resektat herausgenommen werden, und das Bergebeutelinstrument zumindest für denselben Patienten bei derselben Operation wiederverwendet werden.

Die Erfindung bietet also den Vorteil, für einen mit Fluid, insbesondere Flüssigkeit, gefüllten Einsatzort konzipiert und entworfen zu sein. Falls mittels des Bergebeutelinstruments der Bergebeutel zugeschoben beziehungsweise geschlossen wird, kann eine enthaltene Flüssigkeit über die Abflusslöcher besonders vorteilhaft abfließen. Auch in einer gasgefüllten Umgebung ist die Erfindung vorteilhaft, da ein Platzen des Bergebeutels beispielsweise beim Herausziehen des Bergebeutels vermieden werden kann. Gewebe kann in dem Bergebeutel sicher zurückgehalten werden, und beim Schließen des Bergebeutels tritt kein Trampolineffekt oder Platzen des Bergebeutels auf, da bei einer Verkleinerung des Volumens des Bergebeutels das Fluid nicht durch die Fangöffnung, sondern durch die Abflusslöcher nach außen treten kann. Somit ist es dem Benutzer des Bergebeutelinstruments möglich, ein Resektat besonders sicher einzufangen und in dem Bergebeutel aus dem Körper des Patienten (nach außen) herauszuziehen, wobei ein Platzen des Bergebeutels vermieden werden kann.

Die Erfindung umfasst weitere Ausgestaltungen, durch die sich zusätzliche Vorteile ergeben.

Gemäß einer vorteilhaften Ausgestaltung ist vorgesehen, dass der Bergebeutel eine Vielzahl von Durchtrittsöffnungen aufweist, die um die Fangöffnung herum angeordnet sind und durch die das Halteseil hindurchgeführt ist, um den Bergebeutel im Bereich der Schlinge an dem Halteseil zu halten, wobei insbesondere der Bergebeutel an seinem distalen Ende einen schlauchförmigen Schlauchabschnitt aufweist, durch den das Halteseil hindurchgeführt ist. Mit anderen Worten, das Halteseil ist wechselseitig von innen nach außen beziehungsweise von außen nach innen durch die Durchtrittsöffnungen hindurchgeführt und umläuft dabei die Fangöffnung. An dem distalen Ende des Bergebeutels, das gleichzeitig das distale Ende des Bergebeutelinstruments ist, ist der Schlauchabschnitt ausgebildet, durch den das Halteseil hindurchgeführt ist, insbesondere tabaksbeutelartig hindurchgeführt ist. Im Bereich der Durchtrittsöffnungen ist das Halteseil an dem Bergebeutel nicht befestigt, sodass das Beutelmaterial auf dem Halteseil verschiebbar ist. In dem Schlauchabschnitt, der an dem distalen Ende des Bergebeutels in Umfangsrichtung der Fangöffnung verläuft, ist das Halteseil mit dem Bergebeutel fest verbunden, insbesondere durch eine Klebung verbunden, sodass das Halteseil sich gegenüber dem Bergebeutel nicht verschieben kann. Dies kann bei einem Aufschieben der Fangöffnung helfen, dass sich die Fangöffnung vollständig öffnen kann, da der Schlauchabschnitt verhindern kann, dass das Halteseil durch eine Lücke zwischen zwei Durchtrittsöffnungen hindurchgeschoben wird ohne den Bergebeutel zu öffnen. Mit anderen Worten, wäre kein Schlauchabschnitt vorhanden, könnte es passieren, dass ein einmal zugezogener Bergebeutel sich nicht mehr öffnen lässt, da bei einem Zurückziehen des Hüllrohrs das Halteseil sich durch alle Durchtrittsöffnungen hindurchschieben könnte, und sich der ziehharmonikaartig zusammengeschobene obere Rand des Bergebeutels als Ganzes auf dem Halteseil zusammen verschieben könnte ohne sich zu entfalten.

Wird das Hüllrohr in Richtung des Schlauchabschnitts, also in Richtung des distalen Endes des Bergebeutelinstruments bewegt, wird die Fangöffnung des Bergebeutels zugezogen beziehungsweise ziehharmonikaartig zusammengeschoben beziehungsweise zugeschoben. Ähnlich wie bei einer Leporellofaltung kann dadurch das Beutelmaterial besonders dicht zusammengeschoben werden, und es kann ein besonders enges Zusammenschieben des Beutelmaterials, und damit ein besonders festes Verschließen der Bergebeutelöffnung beziehungsweise Fangöffnung, erzielt werden.

Der Bergebeutel kann im Bereich der Durchtrittsöffnungen eine Verstärkung aufweisen, sodass die Fangöffnung im Bereich der Durchtrittsöffnungen, das heißt dem oberen Rand des Bergebeutels besonders zugfest ausgebildet ist. Die Verstärkung kann beispielsweise durch ein Aufdoppeln und Verkleben des Beutelmaterials erfolgen.

Gemäß einer weiteren vorteilhaften Ausgestaltung ist vorgesehen, dass die Abflusslöcher in einer, zwei oder mehr Reihen angeordnet sind. Vorteilhafterweise sind in jeder Reihe zwischen drei und sieben Abflusslöcher angeordnet, insbesondere fünf Abflusslöcher angeordnet. Die in Reihen angeordneten Abflusslöcher können von Reihe zu Reihe versetzt sein, um eine besonders reißfeste Bergebeutelstruktur zu erhalten. Insbesondere ist am Beutelgrund kein Abflussloch angeordnet, und die Abflusslöcher können gleichmäßig verteilt sein, sodass auch bei einer Verlegung beziehungsweise Blockierung von einem oder mehreren der Abflusslöcher beispielsweise durch das Resektat eine optimale Abflussmöglichkeit bei einem Zusammenschieben des Bergebeutels gewährleistet ist. Die Abflusslöcher sind vorteilhafterweise näher zu der Fangöffnung als zu dem Beutelgrund angeordnet. Das heißt, dass im Bereich des Beutelgrunds keine Abflusslöcher vorhanden sind. Durch diese Anordnung kann dennoch ein besonders hervorragendes Abfließen des eingeschlossenen Fluids erzielt werden. Ferner kann erreicht werden, dass das eingefangene Resektat beim Herausziehen des Bergebeutelinstruments durch das Endoskop beziehungsweise durch den oder zusammen mit dem Trokar nicht herausgelangt. Da das Resektat nicht beschädigt wird, kann somit ein Cell Seeding sicher vermieden werden.

Gemäß einer weiteren vorteilhaften Ausgestaltung ist vorgesehen, dass die Abflusslöcher einen vorgegebenen Durchmesser im Bereich von 0,1 mm bis 3 mm aufweisen, und insbesondere im Bereich von 0,5 mm bis 1 mm aufweisen, die Fangöffnung in einem geöffneten Zustand einen Durchmesser im Bereich von 10 mm bis 70 mm, und insbesondere im Bereich von 40 mm bis 60 mm aufweist und/oder der Bergebeutel eine Tiefe im Bereich von 30 mm bis 100 mm aufweist, und insbesondere eine Tiefe im Bereich von 40 mm bis 60 mm aufweist.

Ein Durchmesser der Abflusslöcher im Bereich von 0,1 mm bis 3 mm hat sich als vorteilhaft erwiesen, da bei einem kleineren Durchmesser ein Abfließen des Fluids zunehmend behindert wird, und bei einem Durchmesser größer als 3 mm mit größerem Durchmesser eine Gefahr zunimmt, dass das Resektat durch die Abflusslöcher hindurchtreten kann. Insbesondere kann der Durchmesser der Abflusslöcher im Bereich von 0,5 mm bis 1 mm liegen, was den Vorteil aufweist, dass eine besonders reißfeste Struktur des Bergebeutels hergestellt werden kann.

Für die Fangöffnung hat sich ein Bereich von 10 mm bis 70 mm, und insbesondere im Bereich von 40 mm bis 60 mm als vorteilhaft erwiesen. Eine kleinere Fangöffnung ist zumeist nicht geeignet, um ein Resektat einzufangen. Bei einer zu großen Fangöffnung ergibt sich eine Gefahr, dass zu viel Beutelmaterial durch den Trokar / das Endoskop hindurchbewegt werden muss, und das Bergebeutelinstrument in einem Zustand, in dem es in den Körper des Patienten eingeführt werden kann, schon einen zu großen Durchmesser aufweist, wodurch eine endoskopische Operation schwieriger werden kann. Daher hat sich ein Fangöffnungsdurchmesser von 40 mm bis 60 mm als besonders gut geeignet herausgestellt. Somit kann sichergestellt werden, dass der Bergebeutel eine geeignete Beuteltiefe und Öffnungsweite aufweisen kann, um große Resektate (bis 5 - 6 cm Größe) zu umschließen.

Für den Bergebeutel ist eine Tiefe von 30 mm bis 100 mm vorteilhaft, und eine Tiefe im Bereich von 40 mm bis 60 mm besonders vorteilhaft. Bei einer Tiefe kleiner als 30 mm kann das maximale Volumen des Bergebeutels zu klein werden, so dass eine Eignung als Bergebeutelinstrument abnimmt, und bei einer Tiefe größer als 100 mm kann eine Menge an Beutelmaterial zu groß werden. Dennoch kann je nach Einsatzzweck und/oder Einsatzort der Bergebeutel auch eine Tiefe von 50 mm oder mehr aufweisen.

Gemäß einer weiteren vorteilhaften Ausgestaltung ist vorgesehen, dass der Rand des Bergebeutels im Bereich der Fangöffnung farblich markiert ist. Insbesondere kann der Rand des Bergebeutels im Bereich der Fangöffnung mit einer dunklen Farbe farblich markiert sein. Dies ermöglicht eine leichtere Navigation des Bergebeutels durch den Benutzer, dem mithilfe einer Kamera des Endoskops eine nur eingeschränkte Sicht zugänglich ist, und es erleichtert eine Handhabung des Bergebeutelinstruments als Manipulator. Insbesondere lässt sich die Fangöffnung des Bergebeutelinstruments als Greifer verwenden, mit dem insbesondere das Resektat teilweise gegriffen werden kann, um es in eine vorteilhafte Position für ein vollständiges Einfangen zu bringen. Hierbei ist eine farbliche Markierung besonders vorteilhaft, da in dem Kamerabild leichter erkennbar ist, wo die Öffnung gelegen ist und wie sie orientiert ist. Ferner lässt sich durch die farblich markierte Fangöffnung ein Öffnen und Schließen im endoskopischen Bild besonders einfach erkennen.

Gemäß einer weiteren vorteilhaften Ausgestaltung ist vorgesehen, dass das Hüllrohr einen vorgegebenen Durchmesser aufweist, sodass bei einem Zuschieben der Fangöffnung das Beutelmaterial nicht in das Hüllrohr gezogen wird. Mit anderen Worten, das Hüllrohr schiebt bei einem Zuschieben der Fangöffnung das Beutelmaterial auf dem Halteseil zusammen und das Hüllrohr weist einen derart kleinen Durchmesser auf, sodass das Beutelmaterial nicht in das Hüllrohr gelangen kann (dessen freier Innendurchmesser hauptsächlich durch das einfach oder doppelt geführte Halteseil belegt ist). Dadurch, dass das Beutelmaterial bei einem Zuziehen des Bergebeutels nicht in das Hüllrohr gezogen wird, ändert sich die Tiefe des Bergebeutels bei dem Zuziehen des Bergebeutels kaum, und das Bergebeutelvolumen wird bei dem Zuziehen des Bergebeutels nicht durch ein Einziehen in das Hüllrohr stark reduziert und somit ist eine Resektat-Ausstoßneigung beziehungsweise ein Trampolineffekt weiter verringert.

Gemäß einer weiteren vorteilhaften Ausgestaltung ist vorgesehen, dass das Halteseil fest an dem Griffabschnitt der Handkinematik befestigt ist und insbesondere eine vorgegebene Länge aufweist. Dadurch kann erreicht werden, dass der Operateur mit dem Bergebeutelinstrument unter Beobachtung durch beispielsweise eine in dem Endoskop angebrachte Kamera das Bergebeutelinstrument als Manipulator einsetzen kann. Beispielsweise ist es möglich, ein in dem Fluid schwimmendes oder befindliches Resektat mit dem Bergebeutel teilweise zu fangen, dann mittels des geschlossenen Bergebeutels das Resektat in Schwerkraftrichtung über dem Bergebeutel anzuordnen und dann den Bergebeutel wieder zu öffnen. Daraufhin kann das Resektat unter Schwerkrafteinfluss in den Bergebeutel hineinfallen, woraufhin der Bergebeutel geschlossen werden kann und schließlich das Resektat aus der Körperhöhle entfernt werden kann. Dadurch, dass das Halteseil eine vorgegebene feste Länge aufweist und mit dem Griffabschnitt der Handkinematik fest verbunden ist, und dadurch, dass das Hüllrohr als bewegliche Schließkomponente eingesetzt und auf dem Halteseil verschoben werden kann, kann das distale Ende des Bergebeutels und damit das distale Ende des Bergebeutelinstruments trotz des Schließvorgangs lokal an demselben Ort gelegen bleiben, und eine Wahrscheinlichkeit, dass ein Resektat davonschwimmt oder weggeblasen wird, ist deutlich verringert.

Gemäß einer weiteren vorteilhaften Ausgestaltung ist vorgesehen, das Hüllrohr durch einen Abdichtstopfen hindurchgeführt ist, der auf dem Hüllrohr entlang der Richtung des Hüllrohrs beweglich angeordnet ist, um bei einer Verwendung mit einem Trokar und/oder Endoskop eine Abdichtung der Arbeitsöffnung des Arbeitskanals des Trokars zu erreichen, in die das Bergebeutelinstrument einsetzbar ist. Mit anderen Worten, das Hüllrohr durchdringt ein universell einsetzbares Aufsteckventil (den Abdichtstopfen), welches ein unkompliziertes Verbinden und leckageverringertes Arbeiten mit dem Endoskop gewährleisten kann. Bergebeutelinstrumente im Stand der Technik können nicht mit dem Arbeitskanal beziehungsweise der "Bridge" des Resektoskops beziehungsweise Zytoskops leckagefrei verbunden werden.

Gemäß einem weiteren Aspekt der Erfindung ist ein Bergebeutelinstrument zum Bergen von reseziertem Gewebe bei einer endoskopischen Resektion bereitstellt, umfassend einen beutelförmigen Bergebeutel mit einem Beutelgrund und einer dem Beutelgrund gegenüberliegenden verschließbaren Fangöffnung, wobei der Bergebeutel eine Vielzahl von Abflusslöchern mit einem vorgegebenen Durchmesser aufweist, die dazu geeignet sind, ein in dem Bergebeutel befindliches Fluid aus dem Bergebeutel nach außen austreten zu lassen, eine Handkinematik mit einem Griffabschnitt und einem Betätigungsabschnitt, wobei der Betätigungsabschnitt von einer Ausgangsposition in eine Endposition bewegbar ist, und ein Hüllrohr, das zwischen dem Bergebeutel und der Handkinematik angeordnet ist, wobei ein distales Ende des Hüllrohrs an dem Bergebeutel befestigt ist und ein proximales Ende des Hüllrohrs an dem Betätigungsabschnitt befestigt ist, einem Halteseil, das an dem Griffabschnitt der Handkinematik befestigt ist, durch das Hüllrohr geführt ist und eine die Fangöffnung umgebende Schlinge ausbildet, wobei der Bergebeutel in Bezug auf das Halteseil zumindest abschnittsweise bewegbar ist, wobei bei einer Bewegung des Betätigungsabschnitts von der Ausgangsposition in die Endposition das Hüllrohr in Richtung des distalen Endes bewegt wird, um die Fangöffnung des Bergebeutels zuzuschieben.

Ferner weist das Bergebeutelinstrument eine Handkinematik mit einem Griffabschnitt und einem Betätigungsabschnitt auf. Eine Handkinematik ist hierbei eine Vorrichtung, die durch einen Benutzer des Bergebeutelinstruments in die Hand genommen werden kann und wobei der Benutzer mittels der Handkinematik durch Betätigung des Betätigungsabschnitts einerseits den Betätigungsabschnitt von einer Ausgangsposition in eine Endposition bewegen kann und andererseits von der Endposition in die Ausgangsposition bewegen kann. Ferner sind auch beliebige Zwischenpositionen zwischen der Ausgangsposition und der Endposition durch den Benutzer anwählbar beziehungsweise erreichbar oder auswählbar, das heißt, der Benutzer kann den Betätigungsabschnitt auf jede Position zwischen der Ausgangsposition und der Endposition einstellen. An dem Betätigungsabschnitt ist das Hüllrohr angebracht beziehungsweise befestigt.

Hierbei ist der Bergebeutel ist an dem distalen Ende des Bergebeutelinstruments angebracht, mithin an dem Ende, das in den Körper des Patienten eingeführt werden kann. Die Handkinematik ist andererseits an dem proximalen Ende angeordnet, mithin an dem Ende, das durch den Benutzer (Chirurgen) des Bergebeutelinstruments bedient wird und immer außerhalb des Körpers des Patienten verbleibt.

Das Bergebeutelinstrument umfasst ferner ein Hüllrohr, das zwischen dem Bergebeutel und der Handkinematik angeordnet ist. Das Hüllrohr kann hierbei beispielsweise eine Länge im Bereich von 30 cm bis 70 cm und insbesondere im Bereich von 40 cm bis 60 cm aufweisen. Ein distales Ende des Hüllrohrs ist beispielsweise durch Kleben oder Kunststoffschweißen mittels einer Lasche des Bergebeutels an dem Bergebeutel befestigt, und ein proximales Ende des Hüllrohrs ist an dem Betätigungsabschnitt befestigt. Wird der Betätigungsabschnitt der Handkinematik durch den Benutzer des Bergebeutelinstruments betätigt, so kann das Hüllrohr je nach Betätigung vorwärts oder rückwärts d. h. in Richtung des distalen Endes des Hüllrohrs (beziehungsweise nach vorne) oder in Richtung des proximalen Endes des Hüllrohrs (beziehungsweise nach hinten) bewegt werden. Das Hüllrohr weist einen Durchmesser auf, der so klein wie möglich ist, um für eine endoskopische Operationstechnik geeignet zu sein. Beispielsweise wird das Bergebeutelinstrument durch einen Trokar in eine Körperhöhle beziehungsweise in die Harnblase eingeführt. Das distale Ende des Bergebeutelinstruments kann somit durch einen Trokar beziehungsweise durch einen Arbeitskanal des Resektoskops eingeführt werden und sollte daher einen geringen Durchmesser aufweisen.

Die Handkinematik kann derart gestaltet sein, dass bei einer Schließbewegung der Hand (oder des/der Finger(s)), die die Handkinematik bedient, das Hüllrohr vorwärtsbewegt wird beziehungsweise in Richtung des distalen Endes des Hüllrohrs bewegt wird. Alternativ kann die Handkinematik derart gestaltet sein, dass bei einer Öffnungsbewegung der Hand (oder des/der Finger(s)) das Hüllrohr in Richtung des distalen Endes des Hüllrohrs bewegt wird. Die Handkinematik kann zudem eine Positionsskala zur Information des Benutzers aufweisen, wobei die Skala von einem Skalenwert, der die Ausgangsposition der Handkinematik repräsentiert, bis zu einem Skalenwert reicht, der die Endposition der Handkinematik repräsentiert, und somit dem Benutzer des Bergebeutelinstruments signalisiert, wie weit geöffnet oder geschlossen die Fangöffnung des Bergebeutels ist. Die Skala kann beispielsweise an dem Griffabschnitt der Handkinematik angeordnet sein und kann eine relative Öffnungsweite der Fangöffnung anzeigen.

Das Bergebeutelinstrument umfasst ebenso das Halteseil. Das Halteseil ist an dem Griffabschnitt der Handkinematik befestigt beziehungsweise daran angebracht, ist durch das Hüllrohr geführt und bildet eine die Fangöffnung umgebende Schlinge aus. Hierbei kann das Halteseil einstückig sein, kann insbesondere eine Litze oder ein einadriges Seil sein, kann insbesondere aus einem körperverträglichen Material ausgebildet sein, kann insbesondere dehnfest sein und kann insbesondere aus Nitinol oder dergleichen ausgebildet sein. Ferner kann das Halteseil ein beschichtetes Halteseil sein, um beispielsweise eine Reibung in dem Hüllrohr zu verringern. Das Halteseil kann zweifach durch das Hüllrohr geführt sein (hin- und zurückgeführt sein), um die die Fangöffnung umgebende Schlinge auszubilden, wobei beide Enden des Halteseils an dem Griffabschnitt der Handkinematik angebracht sind. Wahlweise kann das Halteseil nur einfach durch das Hüllrohr geführt sein, um auf diese Weise den notwendigen kleinsten Durchmesser des Hüllrohres verringern zu können, und kann mit einem Verbindungspunkt im Bereich des distalen Endes des Hüllrohrs mit der die Fangöffnung umgebenden Schlinge verbunden zu sein.

Bei einer Bewegung des Betätigungsabschnitts von der Ausgangsposition in die Endposition wird das Hüllrohr in Bezug auf das Halteseil in Richtung des distalen Endes des Hüllrohrs vorwärtsbewegt, und da das Halteseil an dem Griffabschnitt fest befestigt ist, wird somit (in Bezug auf das Hüllrohr) das Halteseil in das Hüllrohr hineingezogen. Da der Bergebeutel im Bereich der Fangöffnung auf dem Halteseil verschiebbar gehalten ist, wird bei einer Bewegung des Betätigungsabschnitts von der Ausgangsposition in die Endposition der Bergebeutel gardinenartig beziehungsweise ziehharmonikaartig zusammengeschoben und so die Fangöffnung des Bergebeutels zugeschoben. Es ist hierbei wichtig, von einem Zuschieben statt von einem Zuziehen der Fangöffnung zu sprechen, da eine Position des distalen Endes des Bergebeutelinstruments in Bezug auf die Handkinematik sich hierbei kaum verändert.

Das Halteseil ist von dem Griffabschnitt durch das Hüllrohr zu dem Bergebeutel geführt, ist rund um die Fangöffnung geführt, um die Fangöffnung zu umgeben, und ist durch das Hüllrohr zu dem Griffabschnitt zurückgeführt. Hierbei ist das Hüllrohr entlang des Halteseils verschiebbar, um die Fangöffnung zuschieben zu können. Das Halteseil kann durch eine Vielzahl von Durchtrittsöffnungen, vorzugsweise 10 Durchtrittsöffnungen oder mehr, und noch bevorzugter 20 Durchtrittöffnungen oder mehr, hindurchgeführt sein. Eine große Anzahl von Durchtrittsöffnungen ist vorteilhaft, um eine große Zugkraft auf den Beutel übertragen zu können, ohne dass ein Ausreißen der Durchtrittsöffnungen droht. Der Bergebeutel kann hierbei bei einer Draufsicht auf die Fangöffnung in Bezug auf eine Durchmesserrichtung der Fangöffnung symmetrisch insbesondere achsensymmetrisch ausgebildet sein. Die Möglichkeit, die Fangöffnung zuschieben zu können, und die Anordnung des Halteseils in Bezug auf das Hüllrohr und die Fangöffnung des Bergebeutels wird auch als Zuschiebemechanik bezeichnet.

Gemäß einem weiteren Aspekt der Offenbarung ist ein Verfahren zur Herstellung eines Bergebeutels für ein Bergebeutelinstrument gemäß der vorstehenden Beschreibung bereitgestellt, wobei das Verfahren die nachstehenden Schritte aufweist. Zunächst wird der Bergebeutel aus einem Polymermaterial und/oder Kunststoff thermogeformt beziehungsweise tiefgezogen. Es ist besonders bevorzugt, dass der Bergebeutel aus einem hochreißfesten, flüssigkeitsdichten, elastischen und/oder thermoplastischen Kunststoff hergestellt ist. Vorzugsweise ist der Bergebeutel aus Polyurethan hergestellt.

In einem nächsten Schritt werden Abflusslöcher erzeugt, indem beispielsweise die Abflusslöcher gestanzt oder durch Laserschneiden ausgeschnitten werden.

In einem nächsten Schritt können die Durchtrittsöffnungen erzeugt werden. Das Erzeugen der Durchtrittsöffnungen kann wie zuvor durch Stanzen beziehungsweise Laserschneiden oder andere im Stand der Technik bekannte Verfahren erfolgen.

In einem optionalen Schritt kann eine Farbmarkierung am oberen Rand des Bergebeutels angebracht werden. Hierzu kann eine dunkle Farbe auf den oberen Rand des Bergebeutels aufgetragen werden, die einen hohen Hell-/Dunkelkontrast zu dem restlichen Beutelmaterial aufweist. Vorzugsweise kann eine blaue oder schwarze Farbe aufgebracht werden. Dieser Schritt kann auch zu jedem geeigneten Zeitpunkt des vorstehend erläuterten Verfahrens erfolgen, und kann beispielsweise auch vor einem Thermoformen des Bergebeutels erfolgen.

In einem optionalen Schritt kann das Material des Bergebeutels im Bereich der Durchtrittsöffnungen und/oder im Bereich der Abflusslöcher verstärkt werden, um den Beutel reißfester zu gestalten. Das Verstärken kann beispielsweise durch Aufkleben einer weiteren Schicht des Beutelmaterials erfolgen. Vorteilhafterweise kann das Verstärken des Bergebeutelmaterials vor einem Erzeugen der jeweiligen Löcher erfolgen.

Gemäß einem weiteren Aspekt der Erfindung ist ein Verfahren zur Herstellung eines Bergebeutelinstruments zum Bergen von reseziertem Gewebe bei einer endoskopischen Resektion bereitgestellt, wobei das Verfahren die nachstehenden Schritte umfasst. Zunächst wird ein Bergebeutel gemäß der vorstehenden Beschreibung hergestellt. Nachfolgend wird das Hüllrohr an dem Bergebeutel befestigt. Dies kann beispielsweise dadurch erfolgen, dass die Lasche des Bergebeutels an das Hüllrohr angeklebt beziehungsweise mit diesem verschweißt wird. Nachfolgend wird das Halteseil durch das Hüllrohr hindurchgefädelt beziehungsweise hindurchgeführt, durch die Durchtrittsöffnungen des Bergebeutels auf einer ersten Seite der Fangöffnung gardinenartig beziehungsweise abwechselnd von außen nach innen und von innen nach außen hindurchgeführt, durch den Schlauchabschnitt des Bergebeutels tabaksbeutelartig beziehungsweise längs in Umfangsrichtung der Fangöffnung hindurchgeführt, auf der anderen Seite der Fangöffnung durch die Durchtrittsöffnungen des Bergebeutels gardinenartig beziehungsweise abwechselnd von innen nach außen und von außen nach innen hindurchgeführt, um abschließend durch das Hüllrohr zurückgeführt zu werden. Nachfolgend erfolgt ein Befestigen des Bergebeutels an dem Halteseil im Bereich des Schlauchabschnitts, wobei das Befestigen im Bereich des Schlauchabschnitts beispielsweise durch Kleben des Bergebeutels an das Halteseil in dem Schlauchabschnitt erfolgt. Schließlich erfolgt ein Befestigen des Halteseils an dem Griffabschnitt der Handkinematik und ein Befestigen des Hüllrohrs an dem Betätigungsabschnitt der Handkinematik.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar.

Die Erfindung wird nun anhand eines bevorzugten Ausführungsbeispiels sowie unter Bezugnahme auf die Zeichnung näher erläutert. Es zeigen hierbei:
- Fig. 1a: eine schematische Darstellung eines distalen Endes eines Bergebeutelinstruments in einer Seitenansicht;
- Fig. 1b: eine schematische Draufsicht auf das distale Ende des Bergebeutelinstruments in einem geöffneten Zustand;
- Fig. 1c: eine schematische Draufsicht auf das distale Ende des Bergebeutelinstruments in einem halbgeschlossenen Zustand;
- Fig. 2a: eine schematische Darstellung einer Handkinematik des Bergebeutelinstruments in einer Seitenansicht;
- Fig. 2b: eine schematische Darstellung einer Handkinematik gemäß einem weiteren Ausführungsbeispiel in einer Draufsicht;
- Fig. 3a: eine schematische Darstellung einer Zuschiebemechanik in einem weiteren Ausführungsbeispiel in einem geöffneten Zustand;
- Fig. 3b: eine schematische Darstellung einer Zuschiebemechanik in dem weiteren Ausführungsbeispiel in einem geschlossenen Zustand;
- Fig. 4a: ein schematisches Ablaufdiagramm eines Verfahrens zur Herstellung eines Bergebeutels; und
- Fig. 4b: ein schematisches Ablaufdiagramm eines Verfahrens zur Herstellung des Bergebeutelinstruments.

In den Figuren sind gleiche oder funktionsgleiche Elemente mit gleichen Bezugszeichen versehen.

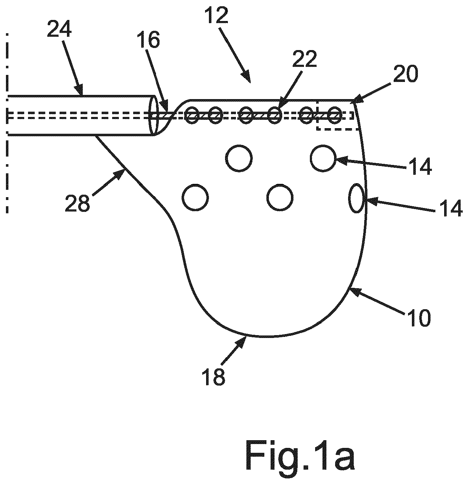

Fig. 1a zeigt eine schematische Darstellung eines distalen Endes eines Bergebeutelinstruments zum Bergen von reseziertem Gewebe bei einer endoskopischen Resektion in einer Seitenansicht. Das Bergebeutelinstrument verfügt über einen beutelförmigen Bergebeutel 10, der mittels einer Verbindungslasche 28 an einem Hüllrohr 24 angebracht ist. Der Bergebeutel 10 ist beutelförmig ausgebildet und weist einen Beutelgrund 18 sowie eine dem Beutelgrund gegenüberliegende verschließbare Fangöffnung 12 auf. Ein Halteseil 16 ist durch das Hüllrohr hindurchgeführt und ist um die Fangöffnung 12 herum durch Durchtrittsöffnungen 22 und einen schlauchartigen Schlauchabschnitt 20 hindurchgeführt, um die Fangöffnung 12 zu umgeben. An dem distalen Ende des Bergebeutelinstruments ist der Bergebeutel 10 angeordnet, und an dem distalen Ende des Bergebeutels 10 ist der Schlauchabschnitt 20 angeordnet, durch den das Halteseil 16 hindurchgeführt ist und an dem das Halteseil 16 mit dem Bergebeutel 10 fest verbunden ist. Das Halteseil 16 kann beispielsweise einstückig ausgebildet sein, kann eine Litze aufweisen oder kann einadrig ausgebildet sein. Ferner kann das Halteseil 16 aus einem körperverträglichen Material ausgebildet sein, kann dehnfest sein und kann beispielsweise aus Nitinol ausgebildet sein. Der Bergebeutel 10 weist in der Nähe der Fangöffnung 12 eine Vielzahl von Abflusslöchern 14 mit vorgegebenem Durchmesser auf, die dazu geeignet sind, ein in dem Bergebeutel befindliches Fluid (Gas und/oder Flüssigkeit) aus dem Bergebeutel 10 nach außen austreten zu lassen. Der Bergebeutel 10 ist aus einem flüssigkeitsundurchlässigen beziehungsweise gasundurchlässigen Material ausgebildet. Die Abflusslöcher 14 weisen jeweils denselben vorgegebenen Durchmesser auf, der sich beispielsweise im Bereich von 0,1 mm bis 3 mm, und insbesondere im Bereich von 0,5 mm bis 1 mm befinden kann.

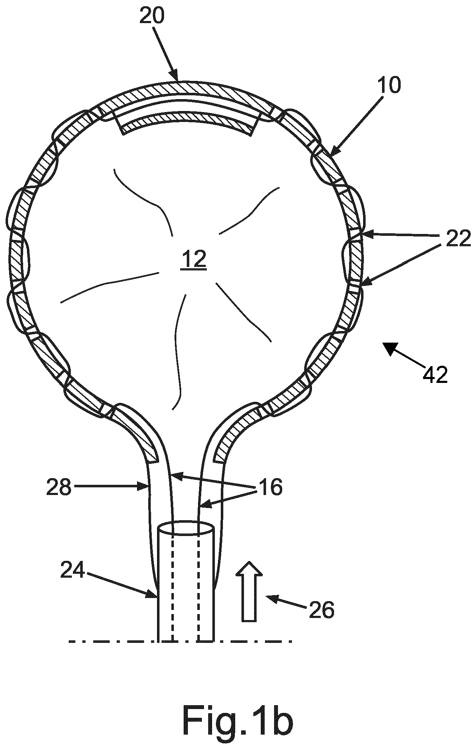

Fig. 1b zeigt eine schematische Draufsicht auf das distale Ende des Bergebeutelinstruments in einem geöffneten Zustand und damit auf die Fangöffnung 12 des Bergebeutelinstruments, wobei sich die Fangöffnung 12 gemäß Fig. 1b in dem geöffneten Zustand befindet. Lediglich der Bergebeutel 10 ist hierbei einer Schnittansicht dargestellt. Es ist zu erkennen, dass das Halteseil 16 abwechselnd von innen nach außen und von außen nach innen durch die Durchtrittsöffnungen 22 hindurchgeführt ist und die Fangöffnung 12 umläuft. Im Bereich des Schlauchabschnitts 20 ist das Halteseil 16 längs beziehungsweise entlang der Umfangsrichtung der Fangöffnung 12 beziehungsweise tabaksbeutelartig durch den Schlauchabschnitt 20 hindurchgeführt. Der Schlauchabschnitt umschließt das Halteseil 16 somit röhrenförmig oder schlauchförmig. Das Halteseil 16 ist in dem Schlauchabschnitt 20 mit dem Bergebeutel 10 fest verbunden. Der Pfeil 26 zeigt eine Zuziehrichtung des Hüllrohrs 24 an, wenn der Betätigungsabschnitt 32 der Handkinematik von einer Ausgangsposition in eine Endposition bewegt wird. Der Bergebeutel 10 ist mittels einer Verbindungslasche 28 an dem Hüllrohr 24 angebracht beziehungsweise befestigt.

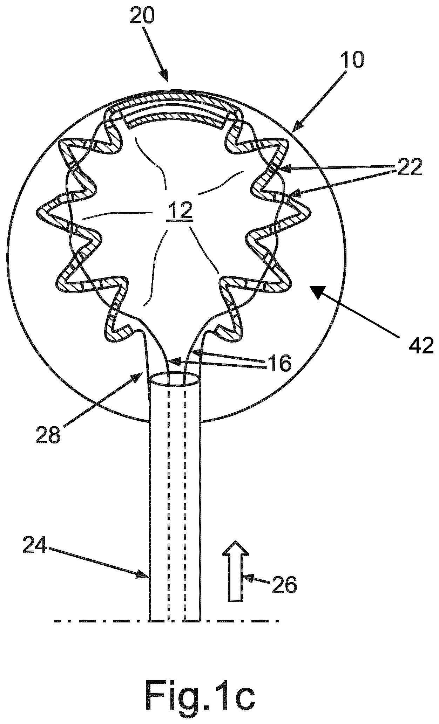

Fig. 1c zeigt eine schematische Draufsicht auf das distale Ende des Bergebeutelinstruments in einem halbgeschlossenen Zustand und damit auf die Fangöffnung 12 des Bergebeutelinstruments, wobei sich die Fangöffnung 12 gemäß Fig. 1c in einem halbgeschlossenen Zustand befindet. Es ist zu erkennen, dass das Hüllrohr 24 entlang der Zuziehrichtung 26 in Richtung des distalen Endes beziehungsweise des Schlauchabschnitts 20 des Bergebeutels 10 vorgeschoben ist, wodurch das Halteseil 16 in Bezug auf das Hüllrohr 24 in das Hüllrohr 24 eingezogen ist, beziehungsweise das Hüllrohr über das Halteseil 16 geschoben ist. Die Fangöffnung 12 ist hierdurch im Gegensatz zu dem geöffneten Zustand gemäß Fig. 1a (in Bezug auf einen Durchmesser und/oder eine Öffnungsfläche der Fangöffnung 12) verkleinert, und der Bergebeutel 10 ist gardinenartig beziehungsweise ziehharmonikaartig auf dem Halteseil 16 zusammengeschoben beziehungsweise aufgeschoben. Durch das Zusammenschieben der Fangöffnung 12 verkleinert sich das Volumen des Bergebeutels 10 in geringem Maße, sodass jedoch eine enthaltene Flüssigkeit teilweise herausgedrückt werden kann. Mittels der Abflusslöcher 14 kann erreicht werden, dass das enthaltene Fluid durch die Fangöffnung 12 nur in geringem Maße herausgedrückt wird (Trampolineffekt).

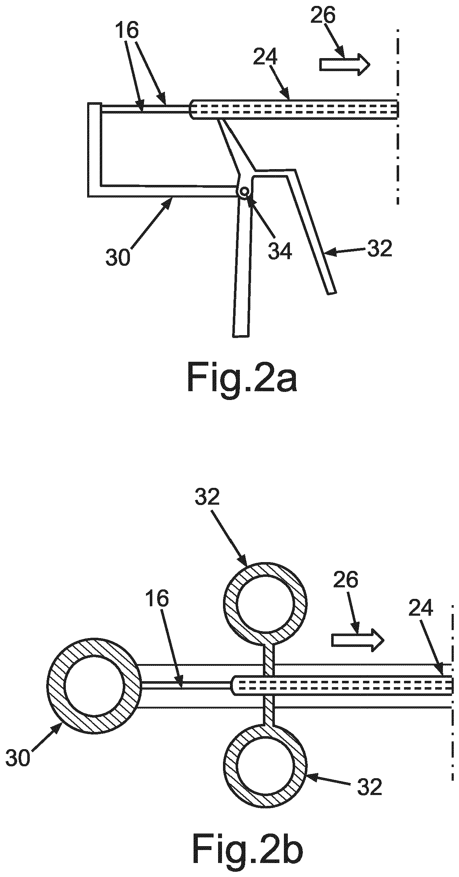

Fig. 2a zeigt eine schematische Darstellung einer Handkinematik des Bergebeutelinstruments in einem Ausführungsbeispiel in einer Seitenansicht, wobei die Handkinematik einen Griffabschnitt 30 (Handstück) und einen Betätigungsabschnitt 32 aufweist. Der Betätigungsabschnitt 32 ist bei diesem Ausführungsbeispiel über eine Drehachse 34 mit dem Griffabschnitt 30 verbunden. Das Halteseil 16 beziehungsweise die zwei Enden des Halteseil 16 sind mit dem Betätigungsabschnitt 32 fest verbunden. Das Hüllrohr 24 ist mit dem Betätigungsabschnitt 32 fest verbunden. Bei einer Betätigung des Betätigungsabschnitts 32 wird das Hüllrohr 24 in Richtung der Zuziehrichtung 26 in Richtung des distalen Endes des Bergebeutelinstruments verschoben. Hierdurch kann die Fangöffnung 12 zugeschoben werden.

Fig. 2b zeigt eine schematische Darstellung einer Handkinematik gemäß einem weiteren Ausführungsbeispiel in einer Draufsicht. Hierbei ist ein Griffabschnitt 30 beispielsweise für einen Daumen des Benutzers ausgebildet, sodass ein Benutzer die Handkinematik dadurch greifen kann, dass er den Zeigefinger und den Mittelfinger durch den Betätigungsabschnitt 32 und seinen Daumen durch den Griffabschnitt 30 steckt. Durch eine Öffnungsbewegung der Hand kann nun der Betätigungsabschnitt 32 in Richtung der Zuziehrichtung 26 in Richtung des distalen Endes des Bergebeutelinstruments bewegt werden. Damit bewegt sich das Hüllrohr 24 in Richtung des Bergebeutels 10, und die Fangöffnung 12 kann somit zugeschoben werden.

Es ist zu beachten, dass die Handkinematik gemäß Fig. 2a den Bergebeutel bei einer schließenden Handbewegung schließt und die Handkinematik gemäß Fig. 2b den Bergebeutel 10 bei einer öffnenden Handbewegung schließt. Somit kann eine bessere und intuitivere Bedienbarkeit des Bergebeutelinstruments erreicht werden.

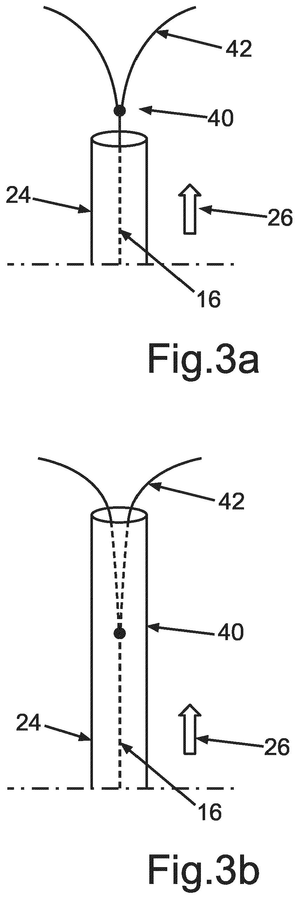

Fig. 3a zeigt eine schematische Darstellung einer Zuschiebemechanik in einem weiteren Ausführungsbeispiel in einem geöffneten Zustand und Fig. 3b zeigt eine schematische Darstellung der Zuschiebemechanik gemäß dem weiteren Ausführungsbeispiel in einem geschlossenen Zustand. Bei diesem Ausführungsbeispiel ist das Halteseil 16 nur einfach durch das Hüllrohr 24 geführt, wobei ein Verbindungspunkt 40 das Halteseil 16, das durch das Hüllrohr 24 geführt ist, mit der Schlinge 42 verbindet, die die Fangöffnung 12 (wie zuvor das Halteseil 16) als Schlinge umgibt. Wird das Hüllrohr 24 in Richtung der Zuziehrichtung 26 in Richtung des distalen Endes des Bergebeutelinstruments bewegt, ist gemäß Fig. 3b ersichtlich, dass der Verbindungspunkt 40 in das Hüllrohr 24 hineingezogen wird, wodurch sich wiederum die Schlinge des Halteseils 16 um die Fangöffnung 12 verkleinert. In den Fig. 3a und 3b sind der Bergebeutel 10 und weitere Einzelheiten des Bergebeutelinstruments nicht gezeigt. Dieses Ausführungsbeispiel weist den Vorteil auf, dass das Hüllrohr 24 in einem großen Bereich dünner ausgeführt und so eine Belastung durch das Bergebeutelinstrument für den Patienten verringert werden kann.

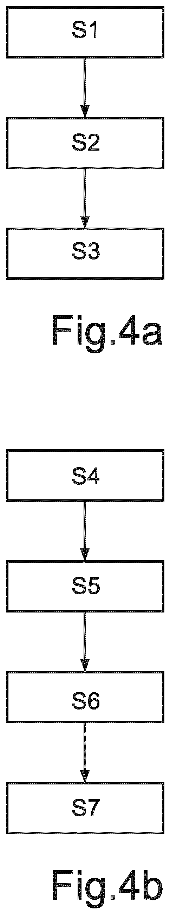

Fig. 4a zeigt ein schematisches Ablaufdiagramm eines Verfahrens zur Herstellung eines Bergebeutels 10 für ein Bergebeutelinstrument gemäß der vorstehenden Beschreibung. In einem Schritt S1 wird eine Grundform des Bergebeutels 10 aus einem Polymermaterial und/oder stabilen Kunststoff durch Thermoformen und/oder Tiefziehen ausgebildet. Es ist besonders bevorzugt, dass das Beutelmaterial aus einem hochreißfesten, flüssigkeitsdichten, elastischen und/oder thermoplastischen Kunststoff ausgebildet wird. Vorzugsweise wird der Bergebeutel 10 aus Polyurethan hergestellt. In einem Schritt S2 werden die Abflusslöcher 14 beispielsweise durch Laserschneiden oder Stanzen in den thermogeformten Bergebeutel eingebracht beziehungsweise erzeugt. In einem Schritt S3 werden die Durchtrittsöffnungen 22 beispielsweise durch Laserschneiden oder Stanzen in den thermogeformten Bergebeutel eingebracht beziehungsweise erzeugt.

In einem vorliegend nicht dargestellten Schritt kann optional im Bereich der Durchtrittsöffnungen 22 das Material des Bergebeutels 10 verstärkt werden. Das Verstärken des Materials des Bergebeutels 10 kann beispielsweise dadurch erfolgen, dass das Material mit einer weiteren Lage desselben Materials verklebt wird. **In** einem weiteren vorliegend nicht dargestellten Schritt kann optional eine Farbmarkierung am oberen Rand des Bergebeutels 10 angebracht werden. Diese beiden optionalen Schritte können vor oder nach jedem der vorgenannten anderen Verfahrensschritte durchgeführt werden.

In Fig. 4b ist ein schematisches Ablaufdiagramm eines Verfahrens zur Herstellung des Bergebeutelinstruments zum Bergen von reseziertem Gewebe bei einer endoskopischen Resektion dargestellt. In einem Schritt S4 erfolgt ein Herstellen eines Bergebeutels 10 gemäß dem vorstehenden Verfahren. In einem Schritt S5 erfolgt ein Befestigen des Hüllrohrs 24 an dem Bergebeutel 10. Hierbei wird beispielsweise die Verbindungslasche 28 mit des Hüllrohrs 24 verklebt beziehungsweise verschweißt (Kunststoffschweißen). In einem Schritt S6 erfolgt ein Durchfädeln des Halteseils 16 durch das Hüllrohr 24 und den Bergebeutel 10. Hierbei wird das Halteseil 16 durch die Durchtrittsöffnungen 22 gardinenartig (abwechselnd von innen nach außen und von außen nach innen) hindurchgeführt, wird entlang der Umfangsrichtung der Fangöffnung 12 längs durch den Schlauchabschnitt 20 des Bergebeutels 10 hindurchgeführt und wird nachfolgend durch die weiteren Durchtrittsöffnungen 22 gardinenartig hindurchgefädelt. Nachfolgend wird das Halteseil 16 durch das Hüllrohr 24 zurückgeführt. In einem Schritt S7 erfolgt ein Befestigen des Halteseils 16 an dem Griffabschnitt 30 der Handkinematik. Ferner erfolgt in diesem Schritt ein Befestigen des Halteseils 16 an dem Bergebeutel 10 im Bereich des Schlauchabschnitts 20 und ein Befestigen des Hüllrohrs 24 an dem Betätigungsabschnitt 32 der Handkinematik.

Es ist ein Bergebeutelinstrument zum Bergen von Gewebe bei einer endoskopischen Resektion bereitstellt, umfassend einen beutelförmigen Bergebeutel 10 mit einer verschließbaren Fangöffnung 12, wobei der Bergebeutel eine Vielzahl von Abflusslöchern 14 mit einem vorgegebenen Durchmesser aufweist, durch die ein in dem Bergebeutel 10 befindliches Fluid aus dem Bergebeutel nach außen austreten kann, eine Handkinematik mit einem Griffabschnitt 30 und einem Betätigungsabschnitt 32 und ein Hüllrohr 24, das zwischen dem Bergebeutel und der Handkinematik angeordnet ist, wobei das Hüllrohr an dem Bergebeutel und an dem Betätigungsabschnitt befestigt ist, einem Halteseil 16, das von dem Griffabschnitt durch das Hüllrohr geführt ist und eine die Fangöffnung umgebende Schlinge 42 ausbildet, wobei der Bergebeutel in Bezug auf das Halteseil zumindest abschnittsweise bewegbar ist, wobei bei einer Bewegung des Betätigungsabschnitts von der Ausgangsposition in die Endposition das Hüllrohr in Richtung des distalen Endes bewegt wird, um die Fangöffnung des Bergebeutels zuzuschieben.

Nachstehend ist ein besonders bevorzugtes Ausführungsbeispiel beschrieben.

Der Bergebeutel des Bergebeutelinstruments umfasst Abfluss- beziehungsweise Drainagelöcher in zwei Ebenen, und umfasst pro Ebene fünf Abfluss- beziehungsweise Drainagelöcher (Perforationslöcher). Am Beutelgrund befindet sich kein Drainageloch, um ein Ausreißen des Beutels zu verhindern. Die Drainagelöcher sind gleichmäßig verteilt, um auch bei Verlegung der Drainageöffnungen durch das Resektat die optimale Drainage beim Komprimieren und Bergen zu gewährleisten. Eine runde Fangöffnung (mit einem Durchmesser von ca. 5 cm) und eine ausreichende Tiefe ermöglicht das Aufnehmen und Umschließen des Resektats.

Der Bergebeutel wird nicht wie herkömmliche Beutel mit der Schlinge beziehungsweise dem Halteseil verklebt. Der Bergebeutel des Bergebeutelinstruments wird im Bereich der Fangöffnung mit verstärkten Durchtrittsöffnungen ausgestattet, durch welche das Halteseil gardinenartig hindurchgenäht wird. Eine hohe Anzahl an Durchtrittsöffnungen (Ösen) ermöglicht es, hohen Zugkräften beim Bergen des Resektats standzuhalten.

Der Bergebeutel des Bergebeutelinstruments wird aufgefädelt. Beim Schließen wird das Beutelmaterial nicht in den Tubus beziehungsweise das Hüllrohr gezogen. Die Tiefe des Beutels ändert sich somit nicht. Das Beutelvolumen wird somit im Bereich der Fangöffnung nur wenig reduziert und verursacht somit ein deutlich verringertes Herausstoßen des Resektats.

Die Beutelschlinge beziehungsweise das Halteseil ist bei dem Bergebeutelinstrument fest mit dem Griff des Instrumentes verbunden. Das Hüllrohr (Tubus) wird als bewegliche Schließkomponente eingesetzt und kann verfahren beziehungsweise bewegt werden. Somit bleibt die Öffnung des Bergebeutels trotz des Schließvorgangs lokal stabil positioniert und eine Wahrscheinlichkeit, dass das Resektat davonschwimmt, ist deutlich reduziert.

Die Fangöffnung (Schlinge) des Bergebeutels eignet sich sehr gut, um beispielsweise ein zum Teil schwebendes Resektat zu greifen und festzuhalten, da das Instrument über die besagte integrierte Fangöffnung verfügt. Der Bergebeutel ist im Bereich der Fangöffnung (Beutelöffnung) farbig markiert und lässt ein Öffnen und Schließen im Kamerabild des Endoskops eindeutig ersichtlich werden. Im (fast) geschlossenen (zugegriffenen) Zustand kann das Resektat angehoben und festgehalten werden. Durch Rotation des Griffs der Handkinematik wird der Bergebeutel nach unten ausgerichtet (Fangöffnung zeigt nach oben entgegen der Schwerkraft). Durch ein anschließendes Öffnen des Beutels kann das Resektat in den Beutel hineinfallen. Das Instrument kann danach ganz an die Kameraoptik des Endoskops herangezogen werden, um in den Beutel hineinzuschauen. Der Benutzer kann sich so ideal von einem Einpackzustand des Resektats überzeugen.

Auf dem Tubus des Instrumentes befindet sich ein integriertes und universell einsatzbares Aufsteckventil, welches ein unkompliziertes Verbinden und leckageminimiertes Arbeiten gewährleistet.

### BEZUGSZEICHENLISTE:

- 10: Bergebeutel
- 12: Fangöffnung
- 14: Abflussloch
- 16: Halteseil
- 18: Beutelgrund
- 20: Schlauchabschnitt
- 22: Durchtrittsöffnung
- 24: Hüllrohr
- 26: Zuziehrichtung
- 28: Verbindungslasche
- 30: Griffabschnitt
- 32: Betätigungsabschnitt
- 34: Drehachse
- 40: Verbindungspunkt
- 42: Schlinge

## Patentansprüche

1. Bergebeutel (10) zum Bergen von reseziertem Gewebe bei einer
endoskopischen Resektion, für ein Bergebeutelinstrument wobei das Bergebeutelinstrument ein Hüllrohr (24), ein Halteseil (16) und eine Schlinge (42) aufweist,
wobei
- der Bergebeutel (10) beutelförmig mit einem Beutelgrund (18) und einer dem Beutelgrund (18) gegenüberliegenden verschließbaren Fangöffnung (12) ausgebildet ist,
- der Bergebeutel (10) eine Vielzahl von Abflusslöchern (14) mit einem vorgegebenen Durchmesser aufweist, die dazu geeignet sind, ein in dem Bergebeutel (10) befindliches Fluid aus dem Bergebeutel (10) nach außen austreten zu lassen, und
- der Bergebeutel (10) an einem proximalen Ende des Bergebeutels (10) eine Verbindungslasche (28) aufweist, die dazu eingerichtet ist, an dem Hüllrohr (24) befestigt zu werden,
**dadurch gekennzeichnet, dass**
- der Bergebeutel (10) eine Vielzahl von Durchtrittsöffnungen (22) aufweist, die um die Fangöffnung (12) herum angeordnet sind und die dazu eingerichtet sind, das Halteseil (16) hindurchzuführen, an dem der Bergebeutel (10) im Bereich der Schlinge (42) haltbar ist, und
- der Bergebeutel (10) an einem dem proximalen Ende gegenüberliegenden distalen Ende einen schlauchförmigen Schlauchabschnitt (20) aufweist, durch den das Halteseil (16) in Umfangsrichtung der Fangöffnung (12) hindurchführbar ist.

2. Bergebeutel nach Anspruch 1, wobei die Abflusslöcher (14) in einer oder zwei Reihen angeordnet sind, und wobei vorteilhafterweise in jeder Reihe zwischen drei und sieben Abflusslöcher (14), insbesondere fünf Abflusslöcher (14) angeordnet sind.

3. Bergebeutel nach einem der vorhergehenden Ansprüche, wobei die Abflusslöcher (14) einen vorgegebenen Durchmesser im Bereich von 0,1 mm bis 3 mm, insbesondere im Bereich von 0,5 mm bis 1 mm, aufweisen,
- die Fangöffnung (12) in einem geöffneten Zustand einen Durchmesser im Bereich von 10 mm bis 70 mm, insbesondere im Bereich von 40 mm bis 60 mm aufweist und/oder
- der Bergebeutel (10) eine Tiefe im Bereich von 30 mm bis 100 mm aufweist, und insbesondere eine Tiefe im Bereich von 40 mm bis 60 mm aufweist.

4. Bergebeutel nach einem der vorhergehenden Ansprüche, wobei der Rand des Bergebeutels (10) im Bereich der Fangöffnung (12) farblich markiert ist.

5. Bergebeutelinstrument mit dem Bergebeutel (10) nach einem der vorhergehenden Ansprüche zum Bergen von reseziertem Gewebe bei einer endoskopischen Resektion, umfassend
- den Bergebeutel (10),
- eine Handkinematik mit einem Griffabschnitt (30) und einem Betätigungsabschnitt (32), wobei der Betätigungsabschnitt (32) von einer Ausgangsposition in eine Endposition bewegbar ist, und
- das Hüllrohr (24), das zwischen dem Bergebeutel (10) und der Handkinematik angeordnet ist, wobei ein distales Ende des Hüllrohrs (24) an dem Bergebeutel (10) befestigt ist und ein proximales Ende des Hüllrohrs (24) an dem Betätigungsabschnitt (32) befestigt ist,
- das Halteseil (16), das an dem Griffabschnitt (30) der Handkinematik befestigt ist, durch das Hüllrohr (24) geführt ist und die die Fangöffnung (12) umgebende Schlinge (42) ausbildet, wobei der Bergebeutel (10) in Bezug auf das Halteseil (16) zumindest abschnittsweise bewegbar ist,
- wobei bei einer Bewegung des Betätigungsabschnitts (32) von der Ausgangsposition in die Endposition das Hüllrohr (24) in Richtung des distalen Endes bewegt wird, um die Fangöffnung (12) des Bergebeutels (10) zuzuschieben.

6. Bergebeutelinstrument nach einem der vorhergehenden Ansprüche, wobei das Hüllrohr (24) einen vorgegebenen Durchmesser aufweist, sodass bei einem Zuschieben der Fangöffnung (12) der Bergebeutel (10) nicht in das Hüllrohr (24) gezogen wird.

7. Bergebeutelinstrument nach einem der vorhergehenden Ansprüche, wobei das Halteseil (16) fest an dem Griffabschnitt (30) der Handkinematik verbunden ist und insbesondere das Halteseil (16) eine vorgegebene Länge aufweist.

8. Bergebeutelinstrument nach einem der vorhergehenden Ansprüche, wobei das Hüllrohr (24) durch einen Abdichtstopfen hindurchgeführt ist, der auf dem Hüllrohr (24) in Richtung des Hüllrohrs (24) beweglich angeordnet ist, um bei einer Verwendung mit einem Trokar und/oder Endoskop eine Abdichtung der Öffnung zu erreichen, in die das Bergebeutelinstrument einsetzbar ist.

9. Verfahren zur Herstellung eines Bergebeutels (10) nach einem der Ansprüche 1 bis 7 mit den Schritten:
- Ausbilden einer Grundform des Bergebeutels (10) aus einem Polymermaterial und/oder Kunststoff durch Thermoformen und/oder Tiefziehen, wobei der Bergebeutel eine Verbindungslasche (28) und einen Schlauchabschnitt (20) aufweist, der der Verbindungslasche (28) gegenüberliegt,
- Erzeugen der Abflusslöcher (14) in dem Beutelmaterial,
- Erzeugen der Durchtrittsöffnungen (22) in dem Beutelmaterial,
- Ausbilden des schlauchartigen Schlauchabschnitts (20), durch den ein Halteseil (16) hindurchführbar ist.

10. Verfahren zur Herstellung eines Bergebeutelinstruments zum Bergen von reseziertem Gewebe bei einer endoskopischen Resektion mit den Schritten:
- Herstellen eines Bergebeutels (10) gemäß dem Verfahren nach dem vorhergehenden Anspruch,
- Befestigen des Hüllrohrs (24) an dem Bergebeutel (10),
- Hindurchführen des Halteseils (16) durch das Hüllrohr (24), Hindurchführen des Halteseils (16) durch die Durchtrittsöffnungen (22) und Hindurchführen des Halteseils (16) durch den Schlauchabschnitt (20) des Bergebeutels (10),
- Befestigen des Bergebeutels (10) an dem Halteseil (16) im Bereich des Schlauchabschnitts (20),
- Befestigen des Halteseils (16) an dem Griffabschnitt (30) der Handkinematik und
- Befestigen des Hüllrohrs (24) an dem Betätigungsabschnitt (32) der Handkinematik.

## Claims

1. A retrieval bag (10) for retrieving resected tissue during an endoscopic resection for a retrieval bag instrument, wherein
- the retrieval bag instrument comprises a sheath tube (24), a retaining string (16) and a sling (42),
- the retrieval bag (10) is bag-shaped with a bag base (18) and a closable capture opening (12) opposite the bag base (18),
- the retrieval bag (10) comprises a plurality of drain holes (14) with a predetermined diameter, which are suitable for allowing a fluid located in the retrieval bag (10) to leak out from the retrieval bag (10) to an outside, and
- the retrieval bag (10) comprises a connecting tongue (28) at a proximal end of the retrieval bag (10), which is adapted to be attached to the sheath tube (24),
**characterized in that**
- the retrieval bag (10) comprises a plurality of passage openings (22), which are arranged around the capture opening (12) and which are adapted to guide through the retaining string (16), at which the retrieval bag (10) can be held in the region of the sling (42), and
- the retrieval bag (10) comprises a tubular tube section (20) at a distal end opposite the proximal end, through which the retaining string (16) can be guided in the circumferential direction of the capture opening (12).

2. The retrieval bag according to claim 1, wherein the drain holes (14) are arranged in one or two rows, and wherein advantageously between three and seven drain holes (14), in particular five drain holes (14), are arranged in each row.

3. The retrieval bag according to any one of the preceding claims, wherein the drain holes (14) comprise a predetermined diameter in the range of 0.1 mm to 3 mm, in particular in the range of 0.5 mm to 1 mm,
- the capture opening (12) in an opened state comprises a diameter in the range of 10 mm to 70 mm, in particular in the range of 40 mm to 60 mm, and/or
- the retrieval bag (10) comprises a depth in the range of 30 mm to 100 mm, and in particular comprises a depth in the range of 40 mm to 60 mm.

4. The retrieval bag according to any one of the preceding claims, wherein the edge of the retrieval bag (10) is color-marked in the region of the capture opening (12).

5. A retrieval bag instrument with the retrieval bag (10) according to any one of the preceding claims for retrieving resected tissue during an endoscopic resection, comprising
- the retrieval bag (10),
- a hand kinematics with a grip section (30) and an actuation section (32), wherein the actuation section (32) is movable from an initial position into an end position, and
- the sheath tube (24), which is arranged between the retrieval bag (10) and the hand kinematics, wherein a distal end of the sheath tube (24) is attached to the retrieval bag (10) and a proximal end of the sheath tube (24) is attached to the actuation section (32),
- the retaining string (16), which is attached to the grip section (30) of the hand kinematics, is guided through the sheath tube (24) and forms the sling (42) surrounding the capture opening (12), wherein the retrieval bag (10) is movable at least in sections with respect to the retaining string (16),
- wherein during a movement of the actuation section (32) from the initial position into the end position the sheath tube (24) is moved in the direction of the distal end in order to push close the capture opening (12) of the retrieval bag (10).

6. The retrieval bag instrument according to any one of the preceding claims, wherein the sheath tube (24) comprises a predetermined diameter, so that during a push closing of the capture opening (12) the retrieval bag (10) is not pulled into the sheath tube (24).

7. The retrieval bag instrument according to any one of the preceding claims, wherein the retaining string (16) is fixedly connected to the grip section (30) of the hand kinematics and in particular the retaining string (16) comprises a predetermined length.

8. The retrieval bag instrument according to any one of the preceding claims, wherein the sheath tube (24) is guided through a sealing plug, which is movably arranged on the sheath tube (24) in the direction of the sheath tube (24) in order to achieve a sealing of the opening into which the retrieval bag instrument is insertable during a use with a trocar and/or an endoscope.

9. A method for manufacturing a retrieval bag (10) according to any one of claims 1 to 7 comprising the steps:
- forming a basic shape of the retrieval bag (10) from a polymer material and/or plastic by thermoforming and/or deep-drawing, wherein the retrieval bag comprises a connecting tongue (28) and a tube section (20), which is opposite the connecting tongue (28),
- creating the drain holes (14) in the bag material,
- creating the passage openings (22) in the bag material and
- forming the tubular tube section (20), through which a retaining string (16) can be guided.

10. A method for manufacturing a retrieval bag instrument for retrieving resected tissue during an endoscopic resection, comprising the steps of:
- manufacturing a retrieval bag (10) according to the method according to the preceding claim,
- attaching the sheath tube (24) to the retrieval bag (10),
- guiding the retaining string (16) through the sheath tube (24), guiding the retaining string (16) through the passage openings (22) and guiding the retaining string (16) through the tube section (20) of the retrieval bag (10),
- attaching the retrieval bag (10) to the retaining string (16) in the region of the tube section (20),
- attaching the retaining string (16) to the grip section (30) of the hand kinematics, and
- attaching the sheath tube (24) to the actuation section (32) of the hand kinematics.

## Revendications

1. Poche de récupération (10) destinée à récupérer du tissu réséqué lors d'une résection endoscopique, pour un instrument pour poche de récupération, l'instrument pour poche de récupération comportant un tube d'enveloppe (24), un câble de retenue (16) et une boucle (42),
- la poche de récupération (10) étant réalisée en forme de poche avec une base de poche (18) et une ouverture de capture (12) pouvant être fermée et opposée à la base de poche (18),
- la poche de récupération (10) comportant une pluralité de trous d'évacuation (14) ayant un diamètre prédéfini, adaptés pour faire sortir hors de la poche de récupération (10), vers l'extérieur, un fluide présent dans la poche de récupération (10), et
- la poche de récupération (10) comportant, à une extrémité proximale de la poche de récupération (10), une languette d'attache (28) conçue pour être fixée sur le tube d'enveloppe (24),
**caractérisée en ce que**
- la poche de récupération (10) comporte une pluralité d'orifices de passage (22) disposés autour de l'ouverture de capture (12) et conçus pour faire passer à travers ceux-ci le câble de retenue (16) sur lequel la poche de récupération (10) peut être maintenue dans la zone de la boucle (42), et
- la poche de récupération (10) comporte, à une extrémité distale opposée à l'extrémité proximale, une section de tube (20) en forme de tube à travers laquelle le câble de retenue (16) peut passer dans une direction circonférentielle de l'ouverture de capture (12).

2. Poche de récupération selon la revendication 1, dans laquelle les trous d'évacuation (14) sont disposés en une ou deux rangées, et dans laquelle, entre trois et sept trous d'évacuation (14), en particulier cinq trous d'évacuation (14), sont ménagés de façon avantageuse dans chaque rangée.

3. Poche de récupération selon l'une des revendications précédentes, dans laquelle les trous d'évacuation (14) ont un diamètre prédéfini dans la plage de 0,1 mm à 3 mm, en particulier dans la plage de 0,5 mm à 1 mm,
- dans un état ouvert, l'ouverture de capture (12) a un diamètre dans la plage de 10 mm à 70 mm, en particulier dans la plage de 40 mm à 60 mm ; et/ou
- la poche de récupération (10) a une profondeur dans la plage de 30 mm à 100 mm et, en particulier, une profondeur dans la plage de 40 mm à 60 mm.

4. Poche de récupération selon l'une des revendications précédentes, dans laquelle le bord de la poche de récupération (10) est marqué en couleur dans la zone d'ouverture de capture (12).

5. Instrument pour poche de récupération muni de la poche de récupération (10) selon l'une des revendications précédentes, destiné à récupérer du tissu réséqué lors d'une résection endoscopique, comportant :
- la poche de récupération (10),
- une cinématique manuelle comportant une section de poignée (30) et une section d'actionnement (32), la section d'actionnement (32) pouvant être déplacée d'une position initiale à une position finale, et
- le tube d'enveloppe (24) disposé entre la poche de récupération (10) et la cinématique manuelle, une extrémité distale du tube d'enveloppe (24) étant fixée sur la poche de récupération (10) et une extrémité proximale du tube d'enveloppe (24) étant fixée sur la section d'actionnement (32),
- le câble de retenue (16) fixé sur la section de poignée (30) de la cinématique manuelle, étant guidé à travers le tube d'enveloppe (24) et formant la boucle (42) entourant l'ouverture de capture (12), la poche de récupération (10) pouvant être déplacée au moins partiellement par rapport au câble de retenue (16),
- lors d'un déplacement de la section d'actionnement (32) de la position initiale à la position finale, le tube d'enveloppe (24) est déplacé en direction de l'extrémité distale afin de pousser l'ouverture de capture (12) de la poche de récupération (10).

6. Instrument pour poche de récupération selon l'une des revendications précédentes, dans lequel le tube d'enveloppe (24) a un diamètre prédéterminé, de telle sorte que lors d'une poussée de l'ouverture de capture (12), la poche de récupération (10) n'est pas tirée dans le tube d'enveloppe (24).

7. Instrument pour poche de récupération selon l'une des revendications précédentes, dans lequel le câble de retenue (16) est fermement attaché à la section de poignée (30) de la cinématique manuelle et le câble de retenue (16) a en particulier une longueur prédéterminée.

8. Instrument pour poche de récupération selon l'une des revendications précédentes, dans lequel le tube d'enveloppe (24) est passé à travers un bouchon d'étanchéité qui est disposé de façon mobile sur le tube d'enveloppe (24) en direction du tube d'enveloppe (24) afin d'obtenir, en cas d'utilisation avec un trocart et/ou un endoscope, une étanchéité de l'ouverture dans laquelle l'instrument pour poche de récupération peut être inséré.

9. Procédé de fabrication d'une poche de récupération (10) selon l'une des revendications 1 à 7 comportant les étapes suivantes :
- réaliser une forme de base de la poche de récupération (10) à partir d'un polymère et/ou d'une matière plastique par thermoformage et/ou emboutissage, la poche de récupération comportant une languette d'attache (28) et une section de tube (20) opposée à la languette d'attache (28),
- produire les trous d'évacuation (14) dans le matériau de la poche,
- produire les ouvertures de passage (22) dans le matériau de la poche,
- réaliser la section de tube (20) en forme de tube à travers laquelle un câble de retenue (16) peut être passé.

10. Procédé de fabrication d'un instrument pour poche de récupération destiné à récupérer du tissu réséqué lors d'une résection endoscopique, comportant les étapes :
- fabriquer une poche de récupération (10) selon le procédé de la revendication précédente,
- fixer le tube d'enveloppe (24) sur la poche de récupération (10),
- faire passer le câble de retenue (16) à travers le tube d'enveloppe (24), faire passer le câble de retenue (16) à travers les orifices de passage (22) et faire passer le câble de retenue (16) à travers la section de tube (20) de la poche de récupération (10),
- fixer la poche de récupération (10) sur le câble de retenue (16) dans la zone de la section de tube (20),
- fixer le câble de retenue (16) sur la section de poignée (30) de la cinématique manuelle et
- fixer le tube d'enveloppe (24) sur la section d'actionnement (32) de la cinématique manuelle.
